# EUROPEAN PATENT SPECIFICATION

(11) **EP 0 419 035 B1**
(45) Date of publication and mention of the grant of the patent: **15.02.1995**
(21) Application number: 90309027.2
(22) Date of filing: 16.08.1990
(51) Int. Cl.: C07D 277/34, C07D 417/12, A61K 31/425

(54) **Thiazolidine dione derivatives**
Thiazolidinedionederivate
Dérivés de thiazolidinedione

(30) Priority: 25.08.1989 GB 8919417
(43) Date of publication of application: 27.03.1991
(73) Proprietor: BEECHAM GROUP PLC, Brentford, Middlesex TW8 9EP (GB)
(72) Inventor: Hindley, Richard Mark, SmithKline Beecham, Epsom, Surrey KT18 5XQ (GB); Southgate, Robert, Betchworth, Surrey RH3 7AJ (GB); Duff, Peter Thomas, The Chemistry Department, Reading RG6 2AH (GB)
(74) Representative: Rutter, Keith, Dr.

(56) References cited:
- EP-A- 0 208 420
- EP-A- 0 257 781
- EP-A- 0 306 228
- EP-A- 0 356 214
- WO-A-89/08652
- Comprehensive Heterocyclic Chemistry, vol. 6, part 4B, p. 235, 270

## Description

This invention relates to certain substituted thiazolidinedione derivatives, to a process for preparing such compounds, to pharmaceutical compositions containing such compounds and to the use of such compounds and compositions in medicine.

European Patent Applications, Publication Numbers 0008203, 0139421, 0155845, 0177353, 0193256, 0207581, 0208420 and 0306228 relate to thiazolidinedione derivatives which are disclosed as having hypoglycaemic and hypolipidaemic activity. Chem. Pharm. Bull 30 (10) 3580-3600 also relates to certain thiazolidinedione derivatives having hypoglycaemic and hypolipidaemic activities.

It has now surprisingly been discovered that certain novel substituted-thiazolidinedione derivatives show improved blood-glucose lowering activity and are therefore of potential use in the treatment and/or prophylaxis of hyperglycaemia and are of particular use in the treatment of Type II diabetes.

These compounds are also indicated to be of potential use for the treatment and/or prophylaxis of other diseases including hyperlipidaemia, hypertension and cardiovascular disease.

Accordingly, the present invention provides a compound of formula (I): 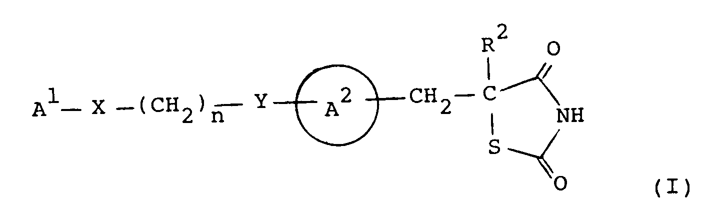
or a tautomeric form thereof and/or a pharmaceutically acceptable salt thereof, and/or a pharmaceutically acceptable solvate thereof, wherein:
A¹ represents a substituted or unsubstituted aromatic heterocyclyl group;
A² represents a benzene ring having in total up to five substituents;
X represents O, S or NR¹ wherein R¹ represents a hydrogen atom, an alkyl group, an acyl group, an aralkyl group, wherein the aryl moiety may be substituted or unsubstituted, or a substituted or unsubstituted aryl group;
Y represents O or S;
R² represents an alkyl, aralkyl or aryl group; and
n represents an integer in the range of from 2 to 6.

Suitable aromatic heterocyclyl groups include substituted or unsubstituted, single or fused ring aromatic heterocyclyl groups comprising up to 4 hetero atoms in each ring selected from oxygen, sulphur or nitrogen.

Favoured aromatic heterocyclyl groups include substituted or unsubstituted single ring aromatic heterocyclyl groups having 4 to 7 ring atoms, preferably 5 or 6 ring atoms.

In particular, the aromatic heterocyclyl group comprises 1, 2 or 3 heteroatoms, especially 1 or 2, selected from oxygen, sulphur or nitrogen.

Suitable values for A¹ when it represents a 5- membered aromatic heterocyclyl group include thiazolyl and oxazolyl, especially oxazolyl.

Suitable values for A¹ when it represents a 6- membered aromatic heterocyclyl group include pyridyl or pyrimidinyl.

Suitably R² represents an alkyl group, in particular a C₁₋₆ alkyl group, for example a methyl group.

Preferably, A¹ represents a moiety of formula (a), (b) or (c): 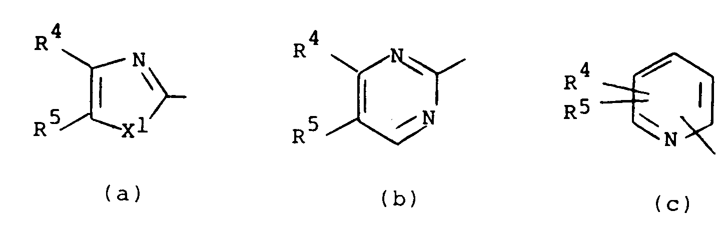
wherein:
R⁴ and R⁵ each independently represents a hydrogen atom, an alkyl group or a substituted or unsubstituted aryl group or when R⁴ and R⁵ are each attached to adjacent carbon atoms, then R⁴ and R⁵ together with the carbon atoms to which they are attached form a benzene ring wherein each carbon atom represented by R⁴ and R⁵ together may be substituted or unsubstituted; and in the moiety of formula (a), X¹ represents oxygen or sulphur.

Aptly, A¹ represents a moiety of the abovedefined formula (a).

Aptly, A¹ represents a moiety of the abovedefined formula (b).

Aptly, A¹ represents a moiety of the abovedefined formula (c).

In one favoured aspect R⁴ and R⁵ together represent a moiety of formula (d): 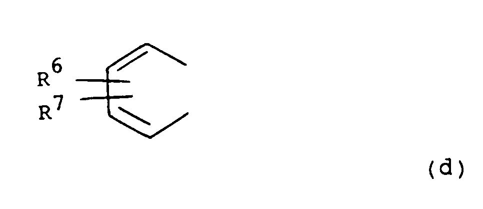
wherein R⁶ and R⁷ each independently represent hydrogen, halogen, substituted or unsubstituted alkyl or alkoxy.

Suitably, R⁶ and R⁷ each independently represent hydrogen, halogen, alkyl or alkoxy.

Favourably, R⁶ represents hydrogen. Favourably, R⁷represents hydrogen.

Preferably, R⁶ and R⁷ both represent hydrogen.

In a further favoured aspect R⁴ and R⁵ each independently represent hydrogen, alkyl or a substituted or unsubstituted phenyl group and more favourably, R⁴ and R⁵ each independently represent hydrogen, alkyl or phenyl.

Preferably, for the moiety of formula (a), R⁴ and R⁵ together represent the moiety of formula (d).

Preferably, for the moieties of formula (b) or (c), especially (c), R⁴ and R⁵ both represent hydrogen.

Suitable substituents for the moiety A² include halogen, substituted or unsubstituted alkyl or alkoxy.

Favourably, A² represents a moiety of formula (e): 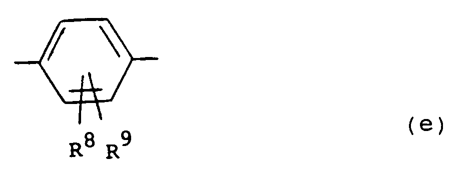
wherein R⁸ and R⁹ each independently represent hydrogen, halogen, substituted or unsubstituted alkyl or alkoxy.

Suitably, R⁸ and R⁹ each independently represent hydrogen, halogen, alkyl or alkoxy.

Preferably, R⁸ and R⁹ each represent hydrogen.

Favourably, X represents oxygen. Favourably, X represents sulphur. Preferably, X represents the above defined moiety NR¹.

Favourably, Y represents O. Favourably Y represents S.

In one preferred aspect the present invention provides a class of compounds, which fall wholly within the scope of formula (I), of formula (II): 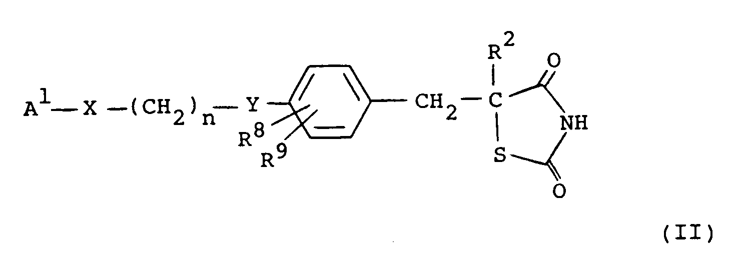
or a tautomeric form thereof, and/or a pharmaceutically acceptable salt thereof and/or a pharmaceutically acceptable solvate thereof, wherein A¹, X, Y, R² and n are as defined in relation to formula (I) and R⁸ and R⁹ are as defined in relation to formula (e).

Suitably, n represents an integer 2, 3 or 4, notably 2 or 3 and especially 2.

Suitably in the moiety NR¹, R¹ represents hydrogen, alkyl, acyl, especially acetyl, or benzyl.

Preferably in the moiety NR¹, R¹ represents a methyl group.

As indicated above a compound of formula (I) may exist in one of several tautomeric forms, all of which are encompassed by the present invention. It will be appreciated that the present invention encompasses all of the isomeric forms of the compounds of formula (I) and the pharmaceutically acceptable salts thereof, including any stereoisomeric forms thereof, whether as individual isomers or as mixtures of isomers.

Suitable substituents for any heterocyclyl group include up to 4 substituents selected from the group consisting of: alkyl, alkoxy, aryl and halogen or any two substituents on adjacent carbon atoms, together with the carbon atoms to which they are attached, may form an aryl group, preferably a benzene ring, and wherein the carbon atoms of the aryl group represented by the said two substituents may themselves be substituted or unsubstituted.

When used herein the term 'aryl' includes phenyl and naphthyl optionally substituted with up to five, preferably up to three, groups selected from halogen, alkyl, phenyl, alkoxy, haloalkyl, hydroxy, amino, nitro, carboxy, alkoxycarbonyl, alkoxycarbonylalkyl, alkylcarbonyloxy, or alkylcarbonyl groups.

When used herein the term 'halogen' refers to fluorine, chlorine, bromine and iodine; preferably chlorine.

When used herein the terms 'alkyl' and 'alkoxy' relate to groups having straight or branched carbon chains,containing up to 12 carbon atoms.

Suitable alkyl groups are C₁₋₁₂ alkyl groups, especially C₁₋₆ alkyl groups e.g. methyl, ethyl, n-propyl, iso-propyl, n-butyl, isobutyl or tert-butyl groups.

Suitable substituents for any alkyl group include those indicated above in relation to the term ''aryl''.

Suitable acyl groups include alkylcarbonyl groups, especially C₁₋₆ alkylcarbonyl groups, for example acetyl groups.

Suitable pharmaceutically acceptable salts include salts of the thiazolidinedione moiety, and, where appropriate, salts of carboxy groups.

Suitable pharmaceutically acceptable salts include metal salts, such as for example aluminium, alkali metal salts such as sodium or potassium, alkaline earth metal salts such as calcium or magnesium and ammonium or substituted ammonium salts, for example those with lower alkylamines such as triethylamine, hydroxyalkylamines such as 2-hydroxyethylamine, bis-(2-hydroxyethyl)amine or tris-(2-hydroxyethyl)amine, cycloalkylamines such as bicyclohexylamine, or with procaine, dibenzylpiperidine, N-benzyl-β-phenethylamine, dehydroabietylamine, N,N′-bisdehydroabietylamine, glucamine, N-methylglucamine or bases of the pyridine type such as pyridine, collidine or quinoline.

In a further aspect the present invention also provides a process for the preparation of a compound of formula (I), or a tautomeric form thereof, and/or a pharmaceutically acceptable salt thereof, and/or a pharmaceutically acceptable hydrate thereof, which process comprises reacting a compound of formula (III): 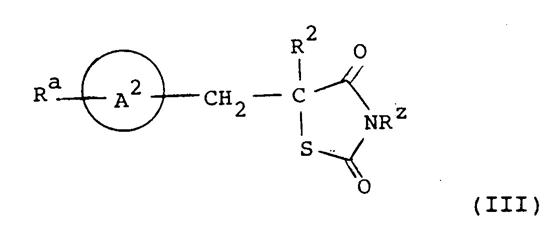
wherein R² and A² are as defined in relation to formula (I), R^{z} is hydrogen or a nitrogen protecting group and R^{a} is a moiety convertible to a moiety of formula (f):

A¹-X-(CH₂)ₙ-Y- (f)

wherein A¹, X, Y and n are as defined in relation to formula (I), with an appropriate reagent capable of converting R^{a} into the said moiety (f) and thereafter, if required, carrying out one or more of the following optional steps:
(i) converting a compound of formula (I) into a further compound of formula (I);
(ii) removing any protecting group;
(iii) preparing a pharmaceutically acceptable salt of the compound of formula (I) and/or a pharmaceutically acceptable solvate thereof.

Suitably, R^{a} represents HX-(CH₂)ₙ-Y- wherein X, Y and n are as defined in relation to formula (I), although Y is preferably -O-.

When R^{a} is HX-(CH₂)ₙ-Y-, an appropriate reagent capable of converting R^{a} into a moiety (f) is a compound of formula (IV):

A¹ - R^{x} (IV)

wherein A¹ is as defined in relation to formula (I) and R^{x} represents a leaving group.

A suitable leaving group R^{x} includes a halogen atom, preferably a chlorine or bromine atom, or a thioalkyl group for example a thiomethyl group.

Suitable values of HX-(CH₂)ₙ-Y- include HO(CH₂)ₙ-O-.

The reaction between the compound of formula (III) and the appropriate reagent may be carried out under conditions suitable to the particular compound of formula (III) and the reagent chosen; thus for example the abovementioned reaction between a compound of formula (III) wherein R^{a} represents HX-(CH₂)ₙ-Y- and the compound of formula (IV), may be carried out in any suitable solvent, for example dimethylformamide, at a temperature which provides a suitable rate of formation of the compound of formula (I), for example at an elevated temperature in the range of from 50°C to 120°C, preferably in the presence of a base such as sodium hydride.

Alternatively, in the reaction between a compound of formula (IV) and a compound of formula (III) wherein R^{a} represents HX-(CH₂)ₙ-Y- and wherein X is NR¹, the reaction may conveniently be carried out in a solvent such as chloroform at a low to medium temperature, for example in the range of from 0° to 30°C, preferably in the presence of a base such as triethylamine.

A compound of formula (III) may be prepared by reacting a compound of formula (V): 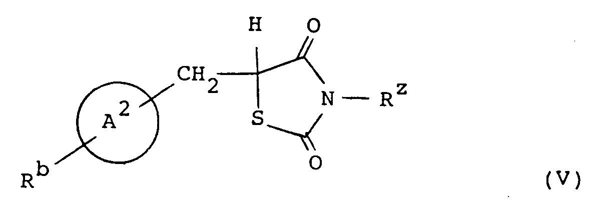
wherein A² is as defined in relation to formula (I), R^{z} is as defined in relation to formula (III) and R^{b} is a moiety R^{a} or a moiety convertible into a moiety R^{a}, with a compound of formula (VI):

R² - R^{c} (VI)

wherein R² is as defined in relation to formula (I) and R^{c} represents a leaving group, such as a halogen atom, for example an iodine atom; and thereafter, if required, converting a moiety R^{b} into a moiety R^{a}.

Preferably, R² in formula (VI) represents alkyl or aralkyl.

The reaction between a compound of formula (V) and (VI) may be carried out in any suitable solvent such as 1,2-dimethoxyethane, at any temperature providing a convenient rate of formation of the required product, suitably at ambient temperature, and preferably in the presence of a base such as an alkali metal base, for example potassium amide in liquid ammonia.

A suitable value for R^{b} is -YR^{d} wherein Y is as defined in relation to formula (I) and R^{d} is a hydrogen atom or, more suitably in the reaction between compounds of formula (V) and (VI), a protecting group such as a benzyl group.

Any conversion of R^{b} into R^{a} may involve a number of steps and thus when R^{d} represents a protecting group the conversion would comprise an appropriate deprotection step, for example a debenzylation step, using standard methods of deprotection, for example debenzylation may be effected by hydrogenolysis.

When R^{a} represents HX-(CH₂)ₙ-Y-, a suitable value for R^{b} is -YH, wherein Y in R^{b} and in the resulting R^{a} have the same value.

The moiety R^{b} may be converted into the moiety R^{a} by any suitable means, for example when R^{b} represents -OH or -SH and R^{a} represents HX-(CH₂)ⁿ-O- or HX-(CH₂)ₙ-S- the appropriate conversion may be carried out by coupling a compound of formula (VA): 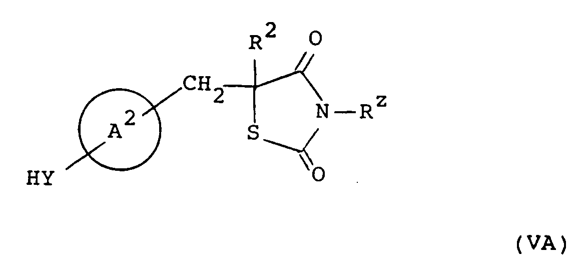
wherein R², Y and A² are as defined in relation to formula (I) and R^{z} is as defined in relation to formula (III) with a compound of formula (VII):

R^{e}-X-(CH₂)ₙ-OR^{f} (VII)

wherein X and n are as defined in relation to formula (I), R^{e} is a protecting group and, when Y in the compound of formula (VA) represents -O-, R^{f} is hydrogen or, when Y in compound (VA) represents -S-, then R^{f} is a tosylate or mesylate group; and thereafter, if required removing any protecting group.

When Y in (VA) is -O- and R^{f} in (VII) is hydrogen, the reaction is generally carried out in the presence of a suitable coupling agent, a suitable coupling agent being provided by diethyl azodicarboxylate and triphenylphosphine. The coupling reaction may be carried out in any suitable solvent at a low to medium temperature, for example in tetrahydrofuran at a temperature in the range of between 0 and 60°C.

When Y in (VA) is -S- and R^{f} in (VII) represents tosylate or mesylate, the reaction between (VA) and (VII) is suitably carried out in an aprotic solvent, such as dimethylformamide, at a low to elevated temperature, for example in the range of from 50°C to 120°C and preferably in the presence of a base such as sodium hydride.

A compound of formula (VII) wherein R^{f} represents a tosylate or a mesylate group may conveniently be prepared from a compound of formula (VII), wherein R^{f} represents hydrogen, using conventional tosylation or mesylation methods.

A suitable protecting group R^{e} is a benzyl group.

A compound of formula (V) may be prepared by reacting a compound of formula (VIII): 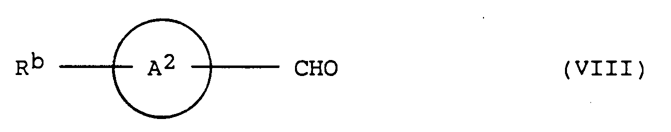
wherein A² is as defined in relation to the compound of formula (I) and R^{b} is as defined in relation to formula (V), with 2,4-thiazolidinedione and reducing the product so formed; and thereafter if necessary converting a moiety R^{b} into a moiety R^{a}.

The reaction between the compound of formula (VIII) and 2,4-thiazolidinedione will of course be carried out under conditions suitable to the nature of the compound of formula (VIII), in general the reaction being carried out in a solvent such as toluene, suitably at an elevated temperature such as the reflux temperature of the solvent and preferably in the presence of a suitable catalyst such as piperidinium acetate or benzoate. Favourably, in the reaction between the compound of formula (VIII) and 2,4-thiazolidinedione, the water produced in the reaction is removed from the reaction mixture, for example by means of a Dean and Stark apparatus.

A suitable reduction method for the abovementioned reduction includes catalytic reduction or the use of a metal/solvent reducing system.

Suitable catalysts for use in the catalytic reduction are palladium on carbon catalysts, preferably a 10% palladium on charcoal catalyst; the reduction being carried out in a solvent, for example dioxan, suitably at ambient temperature.

Suitable metal/solvent reducing systems include magnesium in methanol.

A compound of formula (III) may also be prepared by reacting a compound of formula (IX): 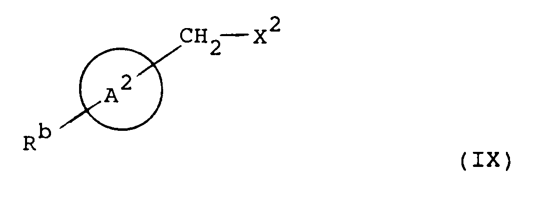
wherein A² and R^{b} are as defined in relation to formula (V) and X² is a halogen atom, with a compound of formula (X): 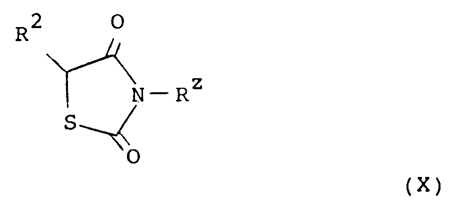
wherein R² is as defined in relation to formula (I) and R^{z} is as defined in relation to formula (III); and thereafter if required, converting a moiety R^{b} into a moiety R^{a}.

The reaction between the compounds of formula (IX) and (X) may be carried out in any suitable solvent, suitably 1,2-dimethoxyethane, at any temperature providing a convenient rate of formation of the required product, suitably at ambient temperature and preferably in the presence of a base such as an alkali metal base, for example potassium amide in liquid ammonia.

Suitably, X² represents a chlorine atom.

A compound of formula (IX) may be prepared from a compound of formula (XI): 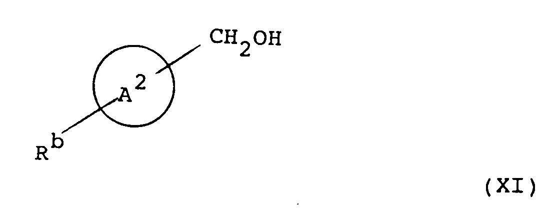
wherein A² and R^{b} are as defined in relation to formula (V), by reaction of the compound of formula (XI) with a halogenating reagent.

Suitable halogenating agents are conventional halogenating agents, for example when X² represents a chlorine atom, a suitable halogenating agent is thionyl chloride.

The conditions for the reaction between the compound of formula (XI) and the halogenating agent will of course depend largely upon the nature of the particular halogenating agent chosen, but the conditions are generally the conventional conditions appropriate to the particular halogenating agent used, for example suitable conditions when the halogenating agent is thionyl chloride involve the use of methylene chloride or chloroform as solvent at a low to medium temperature for example a reaction temperature of between 0 and 30°C.

The compounds of formula (IV), (VI), (VII), (VIII) and (XI) are generally known commercially available compounds or are prepared using methods analogous to those used to prepare such compounds.

The compounds of formula (X) are known compounds or they are prepared according to procedures used to prepare known compounds, for example compounds of formula (X) are disclosed in DE 3045059.

Suitable protecting groups in any of the abovementioned reactions are those used conventionally in the art for example those disclosed in 'Protective Groups in Organic Synthesis', Wiley Interscience, 1981, T.W. Greene. Thus, for example, a suitable nitrogen protecting group is a benzyl group or a benzyloxycarbonyl group and a suitable hydroxyl or thiol protecting group is a benzyl group or a p-methoxybenzyl group.

The methods of formation and removal of such protecting groups are those conventional methods appropriate to the molecule being protected and includes those methods disclosed in the abovementioned 'Protective Groups in Organic Synthesis'.

A compound of formula (I), or a tautomeric form thereof, and/or a pharmaceutically acceptable salt thereof and/or a pharmaceutically acceptable solvate thereof, may also be prepared by reacting a compound of formula (XII): 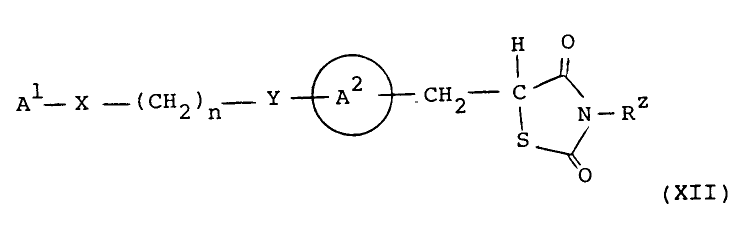
wherein A¹, A², X and Y are as defined in relation to formula (I) and R^{z} is as defined in relation to formula (III), with a compound of the hereinbefore defined formula (VI); and thereafter if required carrying out one or more of the following optional steps:
(i) converting a compound of formula (I) into a further compound of formula (I);
(ii) removing any protecting group;
(iii) preparing a pharmaceutically acceptable salt of a compound of formula (I) and/or a pharmaceutically acceptable solvate thereof.

The reaction between the compounds of formulae (XII) and (VI) may conveniently be carried out under analogous conditions to those described above for the reaction between compounds of formulae (V) and (VI).

A compound of formula (XII) may suitably be prepared by reacting a compound of formula (XIII): 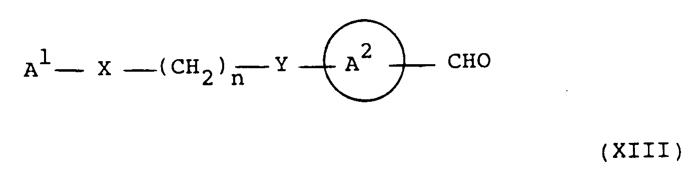
wherein A¹, A², X, Y and n are as defined in respect of the compound of formula (I), with 2,4-thiazolidinedione and thereafter reducing the product so formed.

The reaction between a compound of formula (XIII) and 2,4-thiazolidinedione may suitably be carried out under analogous conditions to those used in the reaction between a compound of formula (VIII) and 2,4-thiazolidinedione.

Suitable reduction methods include those disclosed above for preparing the compounds of formula (V).

A compound of formula (XIII) may be prepared by reacting a compound of formula (XIV): 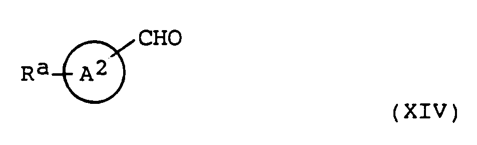
wherein A² is as defined in relation to formula (I) and R^{a} is as defined in relation to formula (III), with an appropriate reagent capable of converting R^{a} to a moiety of the above defined formula (f).

Suitable values for R^{a} include HX-(CH₂)ₙ-Y- wherein X, Y and n are as defined in relation to the compound of formula (I). When R^{a} represents HX-(CH₂)ₙ-Y- the appropriate compound of formula (XIV) may be reacted with a reagent of the abovedefined formula (IV) to provide the required compound of formula (XII).

Suitable reaction conditions for the reaction of the compound of formula (XIV) and the appropriate reagent may include those described above in relation to the preparation of compound (III) with the said appropriate reagent.

Favourably, in the compound of formula (XIV), R^{a} represents a leaving group, especially a fluorine atom. When R^{a} represents a leaving group, preferably a fluorine atom, a particularly appropriate reagent is a compound of formula (XV):

A¹-X-(CH₂)ₙ-YH (XV)

wherein A¹, X, Y and n are as defined in relation to formula (I).

The reaction between the compounds of formulae (XIV) and (XV) may be carried out under any suitable conditions, for example in a solvent such as dimethylformamide or dimethylsulphoxide at an elevated temperature for example in the range of between 100 to 150°C, suitably in the presence of a base such as sodium hydride or potassium carbonate.

Suitably, in the compound of formula (XIV), R^{a} represents a hydroxyl group or a thiol group, and a particularly appropriate reagent is a compound of the abovedefined formula (XV) or a compound of formula (XVA):

A¹-X-(CH₂)ₙ-OR^{g} (XVA)

wherein A¹, X and n are as defined in relation to formula (XV) and R^{g} represents a tosylate or mesylate group.

The reaction between the compound of formula (XIV) wherein R^{a} is a hydroxyl group and the reagent of the above defined formula (XV) may suitably be carried out in an aprotic solvent, such as tetrahydrofuran, at low to medium temperature, for example at ambient temperature, and preferably in the presence of a coupling agent such as that provided by triphenylphosphine and diethyl azodicarboxylate.

The reaction between the compound of formula (XIV), wherein R^{a} is a hydroxyl group or a thiol group, and the reagent of the abovedefined formula (XVA) may be carried out in an aprotic solvent, such as dimethylformamide, at a low to elevated temperature, for example in the range of from 50°C to 120°C and preferably in the presence of a base, such as sodium hydride.

The compound of formula (XVA) may be prepared from the corresponding compound of formula (XV) by reaction with either a tosyl halide or a mesyl halide in a solvent such as pyridine.

The compounds of formula (XIV) are known compounds or they are compounds prepared by methods analogous to those used to prepare known compounds, for example 4-fluorobenzaldehyde and 4-hydroxybenzaldehyde are known commercially available compounds and 4-mercaptobenzaldehyde may be prepared as outlined in Beilstein 8.I.533.

A compound of formula (XII) may also be prepared according to the procedures described in EP0306228.

A compound of formula (I), or a tautomeric form thereof and/or a pharmaceutically acceptable salt thereof and/or a pharmaceutically acceptable solvate thereof, may also be prepared by reacting a compound of formula (XVI): 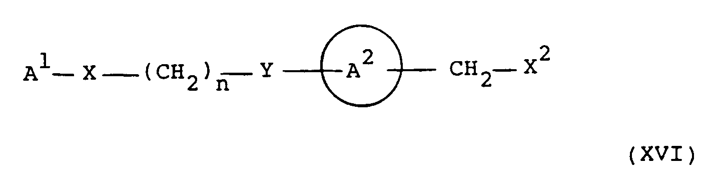
wherein A¹, A², X, Y and n are as defined in relation to formula (I) and X² represents a halogen atom, with a compound of the hereinbefore defined formula (X); and thereafter if required carrying out one or more of the following optional steps:
(i) converting a compound of formula (I) into a further compound of formula (I);
(ii) removing any protecting group;
(iii) preparing a pharmaceutically acceptable salt of a compound of formula (I) and/or a pharmaceutically acceptable solvate thereof.

Suitably X² in the compound of formula (XVI) represents a halogen atom, favourably a chlorine atom.

The reaction between the compounds of formulae (X) and (XVI) may suitably be carried out under analogous conditions to those described above for the reaction between the compounds of formulae (IX) and (X).

A compound of formula (XVI) may be prepared by reacting a compound of formula (XVII): 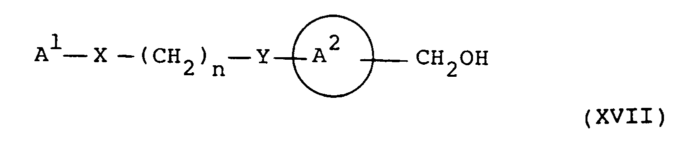
wherein A¹, A², X, Y and n are as defined in relation to formula (I), with a halogenating agent.

Suitable halogenating agents are conventional halogenating agents, for example when X² represents a chlorine atom, a suitable halogenating agent is thionyl chloride.

The conditions for the reaction between the compound of formula (XVII) and the halogenating agent will of course depend largely upon the nature of the particular halogenating agent chosen, but the conditions are generally the conventional conditions appropriate to the particular halogenating agent used, for example suitable conditions when the halogenating agent is thionyl chloride involve the use of methylene chloride or chloroform as solvent at a low to medium temperature for example a reaction temperature of between 0 and 30°C.

A compound of formula (XVII) may be prepared by reacting a compound of formula (XVIII): 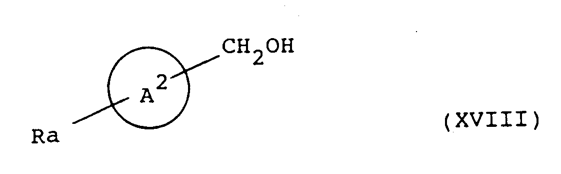
wherein A² and R^{a} are as defined in relation to formula (III), with an appropriate reagent capable of converting a moiety R^{a} into a moiety of the above defined formula (f).

The nature of the moiety R^{a}, the nature of the appropriate reagent and suitable reaction conditions for the reaction between the compound of formula (XVIII) and the appropriate reagent are as described above for the reaction between a compound of formula (III) and the appropriate reagent.

Where necessary a compound of formula (XVIII) may be prepared from a compound of the abovedefined formula (XI), by converting a moiety R^{b} into a moiety R^{a}, using methods hereinbefore described.

The abovementioned conversion of a compound of formula (I) into a further compound of formula (I) includes converting one group R¹ into another group R¹.

The conversion of a compound of formula (I) into a further compound of formula (I) may be carried out by using any appropriate conventional procedure. Thus, suitable conversions of one group R¹ into another group R¹ includes converting a group R¹ which represents hydrogen into a group R¹ which represents an acyl group.

The conversion of a compound of formula (I) wherein R¹ represents hydrogen into a compound of formula (I) wherein R¹ represents acyl may be carried out using any appropriate conventional acylation procedure, such as by treating an appropriately protected compound of formula (I) with an acylating agent. For example acetic anhydride may be used to prepare the compound of formula (I) wherein R¹ is acetyl.

It will be appreciated that in the abovementioned conversion any reactive group in the compound of formula (I) would be protected, according to conventional chemical practice, where necessary.

Where appropriate the isomeric forms of the compounds of formula (I) and the pharmaceutically acceptable salts thereof may be prepared as individual isomers using conventional chemical procedures.

The compounds of formula (III), (V), (IX), (XII), (XVI) and (XVII) are believed to be novel compounds and as such form a further aspect of the invention.

The compounds of formula (XVIII) are known commercially available compounds or they may be prepared according to methods analogous to those used to prepare known compounds.

As mentioned above the compounds of the invention are indicated as having useful therapeutic properties: the present invention accordingly provides a compound of formula (I), or a tautomeric form thereof and/or a pharmaceutically acceptable salt thereof and/or a pharmaceutically acceptable solvate thereof, for use as an active therapeutic substance.

Thus the present invention provides a compound of formula (I), or a tautomeric form thereof and/or a pharmaceutically acceptable salt thereof and/or a pharmaceutically acceptable solvate thereof, for use in the treatment of and/or prophylaxis of hyperglycaemia.

In a further aspect the present invention also provides a compound of formula (I), or a tautomeric form thereof and/or a pharmaceutically acceptable salt thereof and/or a pharmaceutically acceptable solvate thereof, for use in the treatment and/or prophylaxis of hyperlipidaemia.

As indicated hereinbefore the present invention also provides a compound of formula (I) or a tautomeric form thereof and/or a pharmaceutically acceptable salt thereof and/or a pharmaceutically acceptable solvate thereof for use in the treatment of hypertension and cardiovascular disease.

A compound of formula (I), or a tautomeric form thereof and/or a pharmaceutically acceptable salt thereof and/or a pharmaceutically acceptable solvate thereof, may be administered per se or, preferably, as a pharmaceutical composition also comprising a pharmaceutically acceptable carrier.

Accordingly, the present invention also provides a pharmaceutical composition comprising a compound of the general formula (I), or a tautomeric form thereof, or a pharmaceutically acceptable salt thereof, or a pharmaceutically acceptable solvate thereof, and a pharmaceutically acceptable carrier therefor.

As used herein the term 'pharmaceutically acceptable' embraces compounds, compositions and ingredients for both human and veterinary use: for example the term 'pharmaceutically acceptable salt' embraces a veterinarily acceptable salt.

The composition may, if desired, be in the form of a pack accompanied by written or printed instructions for use.

Usually the pharmaceutical compositions of the present invention will be adapted for oral administration, although compositions for administration by other routes, such as by injection and percutaneous absorption are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or other conventional adjuvant.

Typical carriers include, for example, microcrystalline cellulose, starch, sodium starch glycollate, polyvinylpyrrolidone, polyvinylpolypyrrolidone, magnesium stearate, sodium lauryl sulphate or sucrose.

Most suitably the composition will be formulated in unit dose form. Such unit dose will normally contain an amount of the active ingredient in the range of from 0.1 to 1000 mg, more usually 0.1 to 500 mg, and more especially 0.1 to 250 mg.

The present invention further provides a method for the treatment and/or prophylaxis of hyperglycaemia in a human or non-human mammal which comprises administering an effective, non-toxic, amount of a compound of the general formula (I), or a tautomeric form thereof and/or a pharmaceutically acceptable salt thereof and/or a pharmaceutically acceptable solvate thereof to a human or non-human mammal in need thereof.

The present invention further provides a method for the treatment of hyperlipidaemia in a human or non-human mammal, which comprises administering an effective, non-toxic, amount of a compound of formula (I), or a tautomeric form thereof and/or a pharmaceutically acceptable salt thereof and/or a pharmaceutically acceptable solvate thereof, to a human or non-human mammal in need thereof.

Conveniently, the active ingredient may be administered as a pharmaceutical composition hereinbefore defined, and this forms a particular aspect of the present invention.

In the treatment and/or prophylaxis of hyperglycaemia in humans, and/or the treatment and/or prophylaxis of hyperlipidaemia human, the compound of the general formula (I), or a tautomeric form thereof and/or a pharmaceutically acceptable salt thereof and/or a pharmaceutically acceptable solvate thereof, may be taken in doses, such as those described above, one to six times a day in a manner such that the total daily dose for a 70 kg adult will generally be in the range of from 0.1 to 6000 mg, and more usually about 1 to 1500 mg.

In the treatment and/or prophylaxis of hyperglycaemia in non-human mammals, especially dogs, the active ingredient may be adminstered by mouth, usually once or twice a day and in an amount in the range of from about 0.025 mg/kg to 25 mg/kg, for example 0.1 mg/kg to 20 mg/kg. Similar dosage regimens are suitable for the treatment and/or prophylaxis of hyperlipidaemia in non-human mammals.

The dosages regimens for the treatment of hypertension, cardiovascular disease and eating disorders will generally be those mentioned above in relation to hyperglycaemia.

In a further aspect the present invention provides the use of a compound of formula (I), or a tautomeric form thereof and/or a pharmaceutically acceptable salt thereof and/or a pharmaceutically acceptable solvate thereof, for the manufacture of a medicament for the treatment and/or prophylaxis of hyperglycaemia.

The present invention also provides the use of a compound of formula (I), or a tautomeric form thereof and/or a pharmaceutically acceptable salt thereof, and/or a pharmaceutically acceptable solvate thereof, for the manufacture of a medicament for the treatment and/or prophylaxis of hyperlipidaemia, hypertension or cardiovascular disease.

The following Procedures and Examples illustrate the invention.

### Example 1

### 5-(4-[2-(N-methyl-N-(2-benzoxazolyl)amino)ethoxyl benzyl)-5-methyl-2,4-thiazolidinedione.

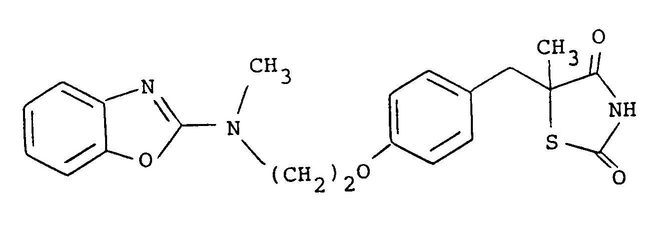

To a stirred solution of potassium amide, prepared from potassium metal (0.69g) in liquid ammonia (150ml), was added a solution of 5-(4-[2-(N-methyl-N-(2-benzoxazolyl)amino)ethoxy]benzyl)-2,4-thiazolidinedione (2.35g, prepared according to procedures described in EP 0306228) in dry 1,2-dimethoxyethane (30ml). The resulting suspension was allowed to stir for 30 minutes. A solution of methyl iodide (2.52g) in dry 1,2-dimethoxyethane (30ml) was added rapidly and the reaction mixture left to stir for one hour, before being neutralized with solid ammonium chloride (2g). The mixture was left open to air and stirred for a further hour, acidified (2M HCl) and left stirring overnight. The mixture was added to water (100 ml), neutralized (2.5M NaOH; sodium bicarbonate solution) and extracted with ethyl acetate (3x150ml). The combined organic extracts were washed with sodium metabisulphite solution (200 ml), brine (200ml), dried (MgS0₄), filtered and evaporated to dryness. The title compound (mp 66-70°C) was obtained as a foam following chromatography on silica-gel of the residual oil in 1% methanol in dichloromethane.
¹H NMR δ (CDCl₃): 1.75 (3H,s); 2.95 (1H,d); 3.25 (1H,d); 3.3 (3H,s); 3.9 (2H,complex); 4.2 (2H,complex); 6.8 (2H,d); 6.95-7.35 (6H,complex); 9.25 (1H,broad s exchanges with D₂0)

### Example 2

### 5-(4-[N-Methyl-N-(2-pyridyl)amino)ethoxy]benzyl)-5-methyl-2,4-thiazolidinedione

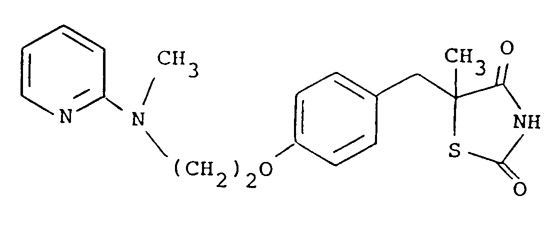

The title compound was obtained as a foam from 5-(4-[N-methyl-N-(2-pyridyl)amino)ethoxy]benzyl)-2,4-thiazol idinedione and iodomethane in an analogous procedure to that used in Example 1.
¹H NMR (CDCl₃:
1.75 (3H,s); 3.15 (3H,s); 2.9-3.8 (2H, complex); 3.6-4.2 (6H, complex); 6.5 (2H, complex); 6.75 (2H, d); 7.10 (2H, d); 7.45 (1H, complex); 8.10 (1H, d); 9.5-12.5 (1H, v. broad s, exchanges with D₂O).

### DEMONSTRATION OF EFFICACY OF COMPOUNDS

### Obese Mice, Oral Glucose Tolerance Test.

C57bl/6 obese (ob/ob) mice were fed on powdered oxoid diet. After at least one week, the mice continued on a powdered oxoid diet or were fed powdered oxoid diet containing the test compound. After 8 days on the supplemented diet all of the mice were fasted for 5 hours prior to receiving an oral load of glucose (3 g/kg). Blood samples for glucose analysis were taken 0, 45, 90 and 135 minutes after glucose administration and the results appear below as the percentage reduction in area under the blood glucose curve where test compound treated groups are compared with the control groups. 7 mice were used for each treatment.

| EXAMPLE NO: | LEVEL IN DIET (»mol kg⁻¹ of DIET) | %REDUCTION IN AREA UNDER BLOOD GLUCOSE CURVE |
|---|---|---|
| 1 | 300 | 40 |
| 2 | 300 | 22 |

### Toxicology

No toxicological effects were indicated for any of the compounds of the invention in any of the abovementioned tests.

## Claims (Claims for the following Contracting State(s): AT, BE, CH, DE, DK, FR, GB, GR, IT, LI, LU, NL, SE)

1. A compound of formula (I): 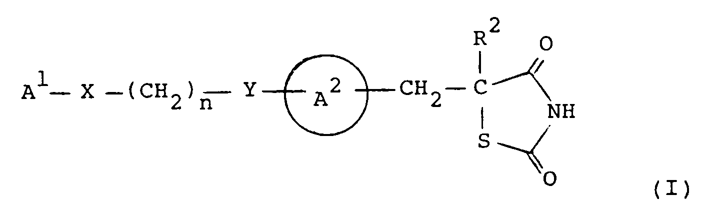 or a tautomeric form thereof and/or a pharmaceutically acceptable salt thereof, and/or a pharmaceutically acceptable solvate thereof, characterised in that:
A¹ represents a substituted or unsubstituted aromatic heterocyclyl group;
A² represents a benzene ring having in total up to five substituents;
X represents O, S or NR¹ wherein R¹ represents a hydrogen atom, an alkyl group, an acyl group, an aralkyl group, wherein the aryl moiety may be substituted or unsubstituted, or a substituted or unsubstituted aryl group;
Y represents O or S;
R² represents an alkyl, aralkyl or aryl group; and
n represents an integer in the range of from 2 to 6.

2. A compound according to claim 1, wherein R² represents an alkyl group.

3. A compound according to claim 1 or claim 2, wherein R² represents a methyl group.

4. A compound according to any one of claims 1 to 3, wherein A¹ represents a moiety of formula (a), (b) or (c): 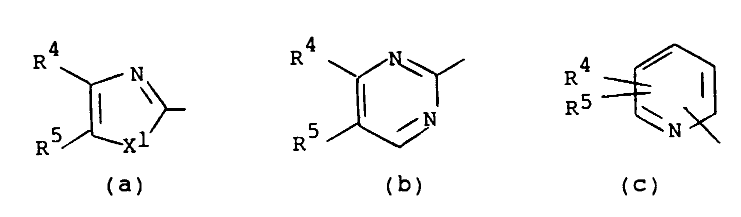 wherein:
R⁴ and R⁵ each independently represents a hydrogen atom, an alkyl group or a substituted or unsubstituted aryl group or when R⁴ and R⁵ are each attached to adjacent carbon atoms, then R⁴ and R⁵ together with the carbon atoms to which they are attached form a benzene ring wherein each carbon atom represented by R⁴ and R⁵ together may be substituted or unsubstituted; and in the moiety of formula (a), X¹ represents oxygen or sulphur.

5. A compound according to claim 4, wherein R⁴ and R⁵ together represent a moiety of formula (d): 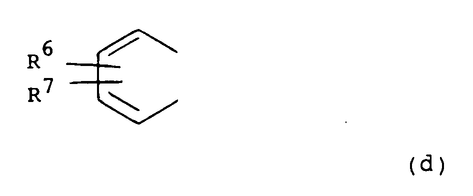 wherein R⁶ and R⁷ each independently represent hydrogen, halogen, substituted or unsubstituted alkyl or alkoxy.

6. A compound according to any one of claims 1 to 5, wherein A² represents a moiety of formula (e): 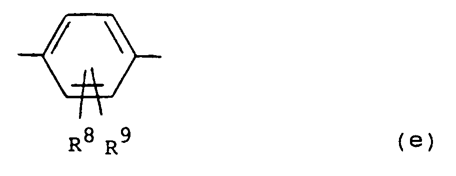 wherein R⁸ and R⁹ each independently represent hydrogen, halogen, substituted or unsubstituted alkyl or alkoxy.

7. A compound according to any one of claims 1 to 6, wherein Y represents O.

8. A compound according to claim 1, being 5-(4-[2-(N-methyl-N-(2-benzoxazolyl)amino)ethoxy] benzyl)-5-methyl-2,4-thiazolidinedione;
or a tautomeric form thereof and/or a pharmaceutically acceptable salt thereof, and/or a pharmaceutically acceptable solvate thereof.

9. A process for the preparation of a compound of formula (I), or a tautomeric form thereof, and/or a pharmaceutically acceptable salt thereof, and/or a pharmaceutically acceptable hydrate thereof, characterised in that the process comprises:
i) reacting a compound of formula (III): 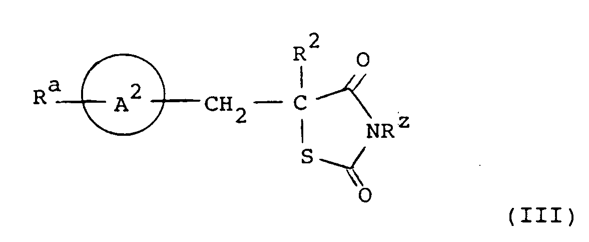 wherein R² and A² are as defined in relation to formula (I) in claim, 1 R^{z} is hydrogen or a nitrogen protecting group and Ra is a moiety convertible to a moiety of formula (f):
A¹-X-(CH₂)ₙ-Y- (f)
wherein A¹, X, Y and n are as defined in relation to formula (I), with an appropriate reagent capable of converting R^{a} into the said moiety (f);
ii) by reacting a compound of formula (XII): 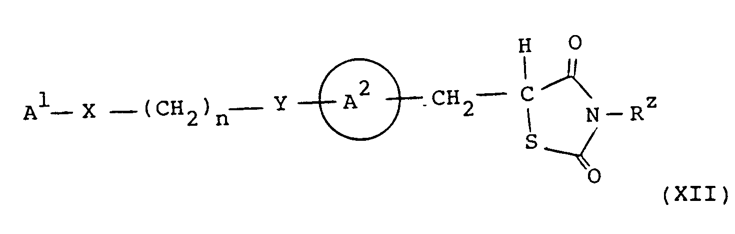 wherein A¹, A², X and Y are as defined in relation to formula (I) in claim 1 and R^{z} is as defined in relation to formula (III), with a compound of formula (VI):
R²-R^{c} (VI)
wherein R² is as defined in relation to formula (I) and R^{c} represents a leaving group; or
iii) by reacting a compound of formula (XVI): 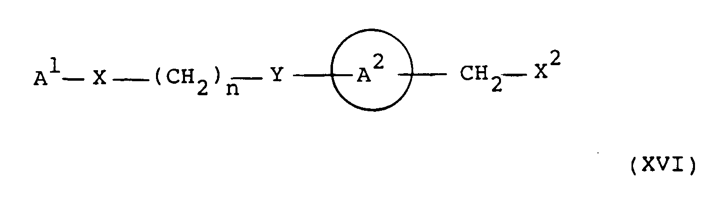 wherein A¹, A², X, Y and n are as defined in relation to formula (I) in claim 1 and X² represents a halogen atom, with a compound of formula (X); 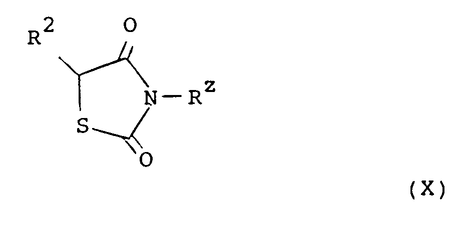 wherein R² is as defined in relation to formula (I) in claim 1 and R^{z} is as defined in relation to formula (III) in reaction i) above; and thereafter if required carrying out one or more of the following optional steps:
(i) converting a compound of formula (I) into a further compound of formula (I);
(ii) removing any protecting group;
(iii) preparing a pharmaceutically acceptable salt of a compound of formula (I) and/or a pharmaceutically acceptable solvate thereof.

10. A pharmaceutical composition comprising a compound of formula (I) according to claim 1, or a tautomeric form thereof or a pharmaceutically acceptable salt thereof or pharmaceutically acceptable solvate thereof, and a pharmaceutically acceptable carrier therefor.

11. A compound of formula (I) according to claim 1, or a tautomeric form thereof and/or a pharmaceutically acceptable salt thereof and/or a pharmaceutically acceptable solvate thereof, for use as an active therapeutic substance.

12. A compound of formula (I) according to claim 1, or a tautomeric form thereof and/or a pharmaceutically acceptable salt thereof and/or a pharmaceutically acceptable solvate thereof, for use in the treatment of and/or prophylaxis of hyperglycaemia.

13. A compound of formula (I) according to claim 1, or a tautomeric form thereof and/or a pharmaceutically acceptable salt thereof and/or a pharmaceutically acceptable solvate thereof, for use in the treatment and/or prophylaxis of hyperlipidaemia and/or hypertension and/or cardiovascular disease.

14. The use of a compound of formula (I) according to claim 1, or a tautomeric form thereof and/or a pharmaceutically acceptable salt thereof and/or a pharmaceutically acceptable solvate thereof, for the manufacture of a medicament for the treatment and/or prophylaxis of hyperglycaemia.

15. The use of a compound of formula (I) according to claim 1, or a tautomeric form thereof and/or a pharmaceutically acceptable salt thereof and/or a pharmaceutically acceptable solvate thereof, for the manufacture of a medicament for the treatment and/or prophylaxis of hyperlipidaemia and/or hypertension and/or cardiovascular disease.

## Claims (Claims for the following Contracting State(s): ES)

1. A process for preparing a compound of formula (I): 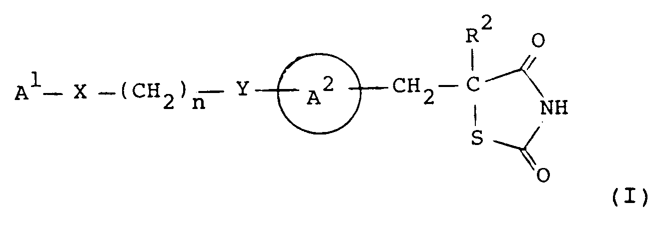 or a tautomeric form thereof and/or a pharmaceutically acceptable salt thereof, and/or a pharmaceutically acceptable solvate thereof, wherein:
A¹ represents a substituted or unsubstituted aromatic heterocyclyl group;
A² represents a benzene ring having in total up to five substituents;
X represents O, S or NR¹ wherein R¹ represents a hydrogen atom, an alkyl group, an acyl group, an aralkyl group, wherein the aryl moiety may be substituted or unsubstituted, or a substituted or unsubstituted aryl group;
Y represents O or S;
R² represents an alkyl, aralkyl or aryl group; and
n represents an integer in the range of from 2 to 6, characterised in that the process comprises:
i) reacting a compound of formula (III): 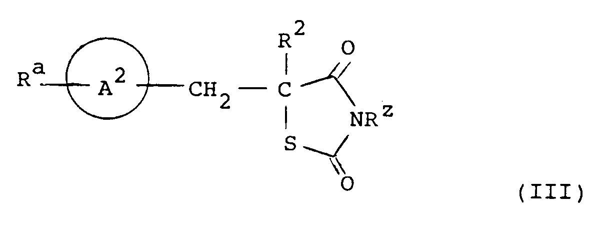 wherein R² and A² are as defined in relation to formula
(I) in claim, 1 R^{z} is hydrogen or a nitrogen protecting group and R^{a} is a moiety convertible to a moiety of formula (f):
A¹-X-(CH₂)ₙ-Y- (f)
wherein A¹, X, Y and n are as defined in relation to formula (I), with an appropriate reagent capable of converting R^{a} into the said moiety (f);
ii) by reacting a compound of formula (XII): 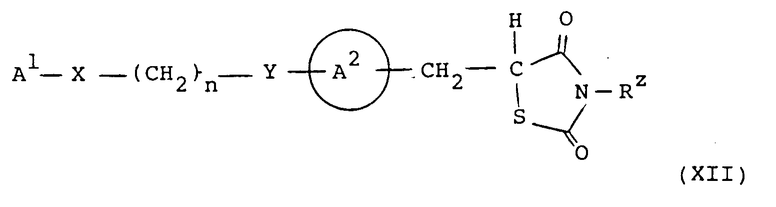 wherein A¹, A², X and Y are as defined in relation to formula (I) in claim 1 and R^{z} is as defined in relation to formula (III), with a compound of formula (VI):
R²-R^{c} (IV)
wherein R² is as defined in relation to formula (I) and R^{c} represents a leaving group; or
iii) by reacting a compound of formula (XVI): 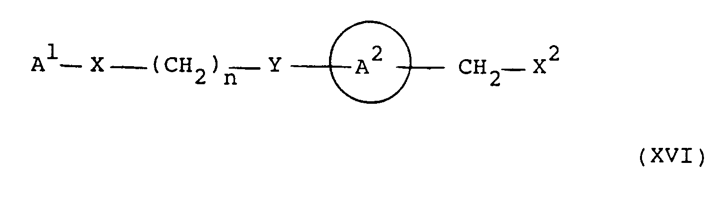 wherein A¹, A², X, Y and n are as defined in relation to formula (I) in claim 1 and X² represents a halogen atom, with a compound of formula (X); 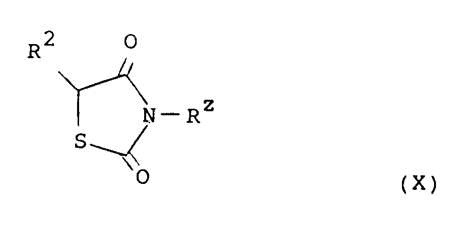 wherein R² is as defined in relation to formula (I) in claim 1 and R^{z} is as defined in relation to formula (III) in reaction i) above; and thereafter if required carrying out one or more of the following optional steps:
(i) converting a compound of formula (I) into a further compound of formula (I);
(ii) removing any protecting group;
(iii) preparing a pharmaceutically acceptable salt of a compound of formula (I) and/or a pharmaceutically acceptable solvate thereof.

2. A process according to claim 1, for preparing a compound wherein R² represents an alkyl group.

3. A process according to claim 1 or claim 2, for preparing a compound wherein R² represents a methyl group.

4. A process according to any one of claims 1 to 3, for preparing a compound wherein A¹ represents a moiety of formula (a), (b) or (c): 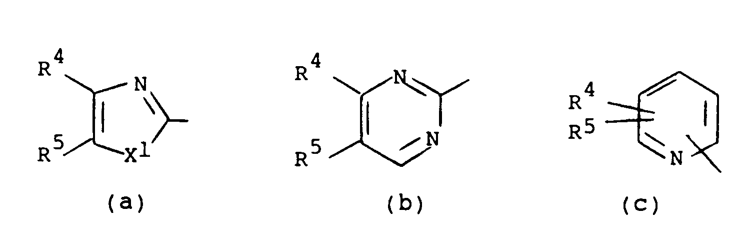 wherein:
R⁴ and R⁵ each independently represents a hydrogen atom, an alkyl group or a substituted or unsubstituted aryl group or when R⁴ and R⁵ are each attached to adjacent carbon atoms, then R⁴ and R⁵ together with the carbon atoms to which they are attached form a benzene ring wherein each carbon atom represented by R⁴ and R⁵ together may be substituted or unsubstituted; and in the moiety of formula (a), X¹ represents oxygen or sulphur.

5. A process according to claim 4, for preparing a compound wherein R⁴ and R⁵ together represent a moiety of formula (d): 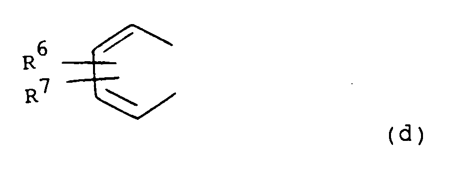 wherein R⁶ and R⁷ each independently represent hydrogen, halogen, substituted or unsubstituted alkyl or alkoxy.

6. A process according to any one of claims 1 to 5, for preparing a compound wherein A² represents a moiety of formula (e) : 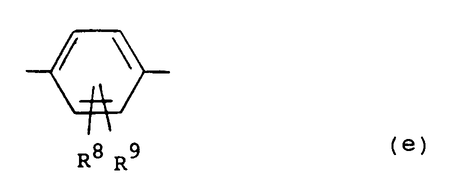 wherein R⁸ and R⁹ each independently represent hydrogen, halogen, substituted or unsubstituted alkyl or alkoxy.

7. A process according to any one of claims 1 to 6, for preparing a compoundwherein Y represents O.

8. A process according to claim 1, for preparing 5-(4-[2-(N-methyl-N-(2-benzoxazolyl)amino)ethoxy] benzyl)-5-methyl-2,4-thiazolidinedione;
or a tautomeric form thereof and/or a pharmaceutically acceptable salt thereof, and/or a pharmaceutically acceptable solvate thereof.

9. The use of a compound of formula (I) according to claim 1, or a tautomeric form thereof and/or a pharmaceutically acceptable salt thereof and/or a pharmaceutically acceptable solvate thereof, for the manufacture of a medicament for the treatment and/or prophylaxis of hyperglycaemia.

10. The use of a compound of formula (I) according to claim 1, or a tautomeric form thereof and/or a pharmaceutically acceptable salt thereof and/or a pharmaceutically acceptable solvate thereof, for the manufacture of a medicament for the treatment and/or prophylaxis of hyperlipidaemia, hypertension or cardiovascular disease.

## Patentansprüche (Patentansprüche für folgende(n) Vertragsstaat(en): AT, BE, CH, DE, DK, FR, GB, GR, IT, LI, LU, NL, SE)

1. Verbindung der Formel (I): 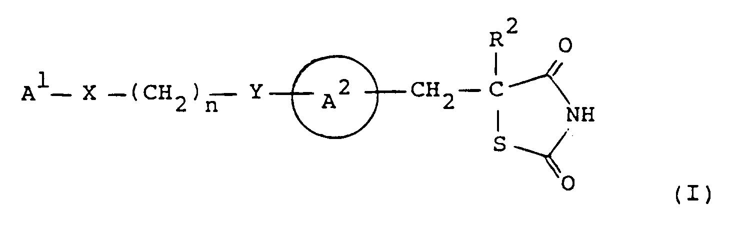 oder eine tautomere Form davon und/oder ein pharmazeutisch verträgliches Salz davon und/oder ein pharmazeutisch verträgliches Solvat davon, dadurch gekennzeichnet, daß:
A¹ einen substituierten oder unsubstituierten aromatischen heterocyclischen Rest darstellt,
A² einen Benzolring mit insgesamt bis zu fünf Substituenten darstellt,
X ein Sauerstoff- oder Schwefelatom oder eine Gruppe NR¹ darstellt, wobei R¹ ein Wasserstoffatom, einen Alkyl-, Acyl-, Aralkylrest, wobei die Aryleinheit substituiert oder unsubstituiert sein kann, oder einen substituierten oder unsubstituierten Arylrest darstellt,
Y ein Sauerstoff- oder Schwefelatom darstellt,
R² einen Alkyl-, Aralkyl- oder Arylrest darstellt, und
n eine ganze Zahl im Bereich von 2 bis 6 darstellt.

2. Verbindung nach Anspruch 1, in der R² einen Alkylrest darstellt.

3. Verbindung nach Anspruch 1 oder Anspruch 2, in der R² eine Methylgruppe darstellt.

4. Verbindung nach einem der Ansprüche 1 bis 3, in der A¹ eine Einheit der Formel (a), (b) oder (c): 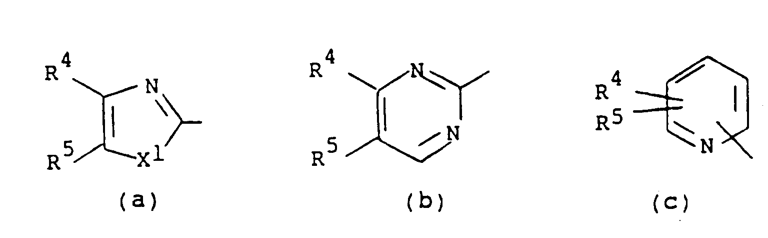 darstellt, in der:
R⁴ und R⁵ jeweils unabhängig ein Wasserstoffatom, einen Alkyl- oder substituierten oder unsubstituierten Arylrest darstellen, oder, wenn R⁴ und R⁵ jeweils an benachbarte Kohlenstoffatome gebunden sind, dann R⁴ und R⁵ zusammen mit den Kohlenstoffatomen, an die sie gebunden sind, einen Benzolring bilden, wobei jedes durch R⁴ und R⁵ zusammen dargestellte Kohlenstoffatom substituiert oder unsubstituiert sein kann, und in der Einheit der Formel (a) X¹ ein Sauerstoff- oder Schwefelatom darstellt.

5. Verbindung nach Anspruch 4, in der R⁴ und R⁵ zusammen eine Einheit der Formel (d): 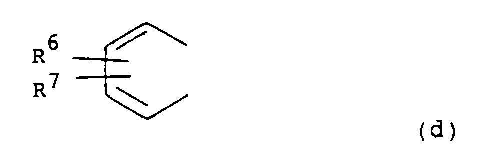 darstellen, in der R⁶ und R⁷ jeweils unabhängig ein Wasserstoff-, Halogenatom, einen substituierten oder unsubstituierten Alkyl- oder Alkoxyrest darstellen.

6. Verbindung nach einem der Ansprüche 1 bis 5, in der A² eine Einheit der Formel (e): 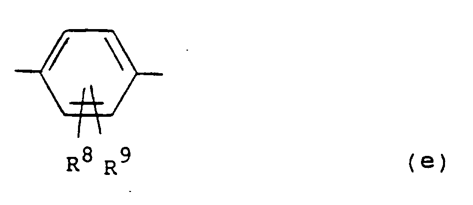 darstellt, in der R⁸ und R⁹ jeweils unabhängig ein Wasserstoff-, Halogenatom, einen substituierten oder unsubstituierten Alkyl- oder Alkoxyrest darstellen.

7. Verbindung nach einem der Ansprüche 1 bis 6, in der Y ein Sauerstoffatom darstellt.

8. Verbindung nach Anspruch 1, nämlich 5-(4-[2-(N-Methyl-N-(2-benzoxazolyl)amino)ethoxy]benzyl)-5-methyl-2,4-thiazolidindion, oder eine tautomere Form davon und/oder ein pharmazeutisch verträgliches Salz davon und/oder ein pharmazeutisch verträgliches Solvat davon.

9. Verfahren zur Herstellung einer Verbindung der Formel (I) oder einer tautomeren Form davon und/oder eines pharmazeutisch verträglichen Salzes davon, und/oder eines pharmazeutisch verträglichen Hydrats davon, dadurch gekennzeichnet, daß das Verfahren umfaßt:
i) Umsetzung einer Verbindung der Formel (III): 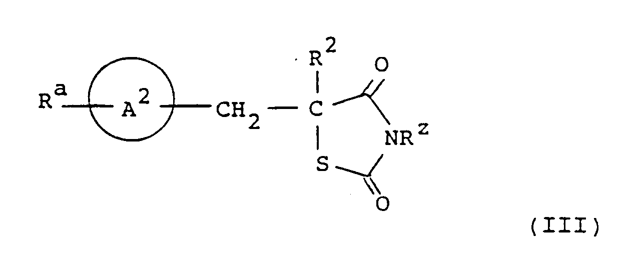 in der R² und A² die in bezug auf Formel (I) in Anspruch 1 angegebene Bedeutung haben, R^{z} ein Wasserstoffatom oder eine Stickstoffschutzgruppe ist und R^{a} eine in eine Einheit der Formel (f) umwandelbare Einheit ist:
A¹-X-(CH₂)ₙ-Y- (f)
wobei A¹, X, Y und n die in bezug auf Formel (I) angegebene Bedeutung haben, mit einem geeigneten Reagens, das dazu fähig ist, R^{a} in die Einheit (f) umzuwandeln;
ii) Umsetzung einer Verbindung der Formel (XII): 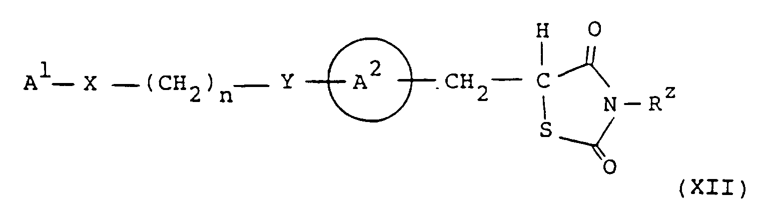 in der A¹, A², X und Y die in bezug auf Formel (I) in Anspruch 1 angegebene Bedeutung haben und R^{z} die in bezug auf Formel (III) angegebene Bedeutung hat, mit einer Verbindung der vorstehenden Formel (VI):
R²-R^{c} (IV)
in der R² wie in Verbindung mit Formel (I) definiert ist und R^{c} eine Abgangsgruppe bedeutet; oder
iii) Umsetzung einer Verbindung der Formel (XVI): 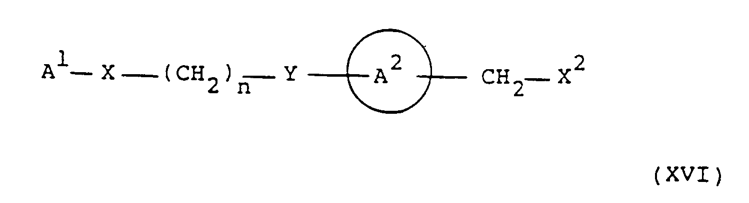 in der A¹, A², X, Y und n die in bezug auf Formel (I) in Anspruch 1 angegebene Bedeutung haben und X² ein Halogenatom darstellt, mit einer Verbindung der Formel (X) 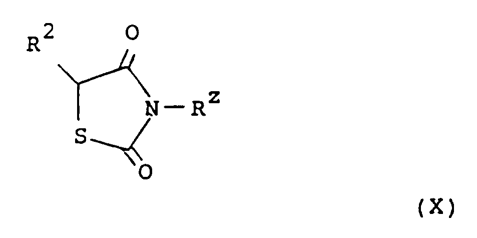 in der R² die in bezug auf Formel (I) in Anspruch 1 angegebene Bedeutung hat und R^{z} die in bezug auf Formel (III) in der vorstehenden Umsetzung i) angegebene Bedeutung hat, und danach falls erforderlich Durchführen eines oder mehrerer der folgenden beliebigen Schritte:
(i) Umwandeln einer Verbindung der Formel (I) in eine weitere Verbindung der Formel (I);
(ii) Abspalten irgendwelcher Schutzgruppen;
(iii) Herstellen eines pharmazeutisch verträglichen Salzes einer Verbindung der Formel (I) und/oder eines pharmazeutisch verträglichen Solvats davon.

10. Arzneimittel, umfassend eine Verbindung der Formel (I) nach Anspruch 1 oder eine tautomere Form davon oder ein pharmazeutisch verträgliches Salz davon oder ein pharmazeutisch verträgliches Solvat davon, und einen pharmazeutisch verträglichen Träger dafür.

11. Verbindung der Formel (I) nach Anspruch 1 oder eine tautomere Form davon und/oder ein pharmazeutisch verträgliches Salz davon und/oder ein pharmazeutisch verträgliches Solvat davon, zur Verwendung als therapeutischer Wirkstoff.

12. Verbindung der Formel (I) nach Anspruch 1 oder eine tautomere Form davon und/oder ein pharmazeutisch verträgliches Salz davon und/oder ein pharmazeutisch verträgliches Solvat davon zur Verwendung bei der Behandlung und/oder Prophylaxe von Hyperglykämie.

13. Verbindung der Formel (I) nach Anspruch 1 oder eine tautomere Form davon und/oder ein pharmazeutisch verträgliches Salz davon und/oder ein pharmazeutisch verträgliches Solvat davon, zur Verwendung bei der Behandlung und/oder Prophylaxe von Hyperlipämie und/oder Bluthochdruck und/oder Herzkreislauferkrankungen.

14. Verwendung einer Verbindung der Formel (I) nach Anspruch 1 oder einer tautomeren Form davon und/oder eines pharmazeutisch verträglichen Salzes davon und/oder eines pharmazeutisch verträglichen Solvates davon zur Herstellung eines Medikaments zur Behandlung und/oder Prophylaxe von Hyperglykämie.

15. Verwendung einer Verbindung der Formel (I) nach Anspruch 1 oder einer tautomeren Form davon und/oder eines pharmazeutisch verträglichen Salzes davon und/oder eines pharmazeutisch verträglichen Solvats davon zur Herstellung eines Medikaments zur Behandlung und/oder Prophylaxe von Hyperlipämie und/oder Bluthochdruck und/oder Herzkreislauferkrankungen.

## Patentansprüche (Patentansprüche für folgende(n) Vertragsstaat(en): ES)

1. Verfahren zur Herstellung einer Verbindung der Formel (I): 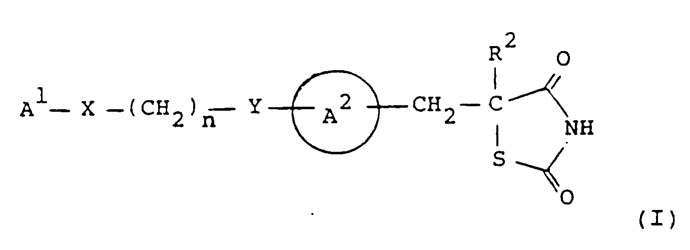 oder einer tautomeren Form davon und/oder eines pharmazeutisch verträglichen Salzes davon und/oder eines pharmazeutisch verträglichen Solvats davon, wobei:
A¹ einen substituierten oder unsubstituierten aromatischen heterocyclischen Rest darstellt,
A² einen Benzolring mit insgesamt bis zu fünf Substituenten darstellt,
X ein Sauerstoff- oder Schwefelatom oder eine Gruppe NR¹ darstellt, wobei R¹ ein Wasserstoffatom, einen Alkyl-, Acyl-, Aralkylrest, wobei die Aryleinheit substituiert oder unsubstituiert sein kann, oder einen substituierten oder unsubstituierten Arylrest darstellt,
Y ein Sauerstoff- oder Schwefelatom darstellt,
R² einen Alkyl-, Aralkyl- oder Arylrest darstellt, und
n eine ganze Zahl im Bereich von 2 bis 6 darstellt, dadurch gekennzeichnet, daß das Verfahren umfaßt:
i) Umsetzung einer Verbindung der Formel (III): 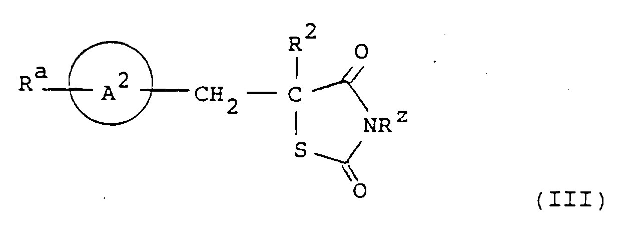 in der R² und A² die in bezug auf Formel (I) in Anspruch 1 angegebene Bedeutung haben, R^{z} ein Wasserstoffatom oder eine Stickstoffschutzgruppe ist und R^{a} eine in eine Einheit der Formel (f) umwandelbare Einheit ist:
A¹-X-(CH₂)ₙ-Y- (f)
wobei A¹, X, Y und n die in bezug auf Formel (I) angegebene Bedeutung haben, mit einem geeigneten Reagens, das dazu fähig ist, R^{a} in die Einheit (f) umzuwandeln;
ii) Umsetzung einer Verbindung der Formel (XII): 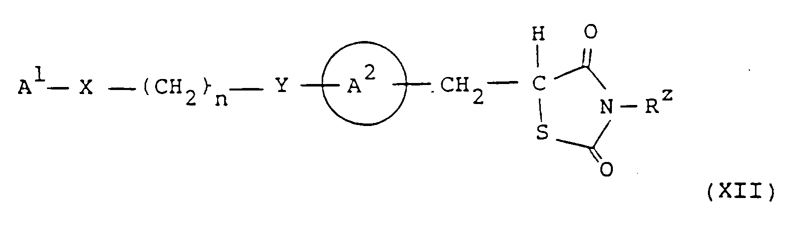 in der A¹, A², X und Y die in bezug auf Formel (I) in Anspruch 1 angegebene Bedeutung haben und R^{z} die in bezug auf Formel (III) angegebene Bedeutung hat, mit einer Verbindung der vorstehenden Formel (VI):
R²-R^{c} (IV)
in der R² wie in Verbindung mit Formel (I) definiert ist und R^{c} eine Abgangsgruppe bedeutet; oder
iii) Umsetzung einer Verbindung der Formel (XVI): 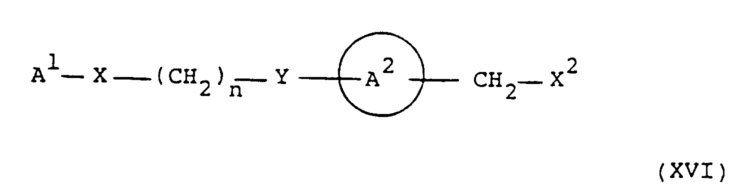 in der A¹, A², X, Y und n die in bezug auf Formel (I) in Anspruch 1 angegebene Bedeutung haben und X² ein Halogenatom darstellt, mit einer Verbindung der Formel (X) 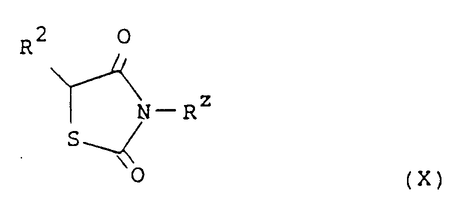 in der R² die in bezug auf Formel (I) in Anspruch 1 angegebene Bedeutung hat und R^{z} die in bezug auf Formel (III) in der vorstehenden Umsetzung i) angegebene Bedeutung hat, und danach falls erforderlich Durchführen eines oder mehrerer der folgenden beliebigen Schritte:
(i) Umwandeln einer Verbindung der Formel (I) in eine weitere Verbindung der Formel (I);
(ii) Abspalten irgendwelcher Schutzgruppen;
(iii) Herstellen eines pharmazeutisch verträglichen Salzes einer Verbindung der Formel (I) und/oder eines pharmazeutisch verträglichen Solvats davon.

2. Verfahren nach Anspruch 1 zur Herstellung einer Verbindung, in der R² einen Alkylrest darstellt.

3. Verfahren nach Anspruch 1 oder Anspruch 2 zur Herstellung einer Verbindung, in der R² eine Methylgruppe darstellt.

4. Verfahren nach einem der Ansprüche 1 bis 3 zur Herstellung einer Verbindung, in der A¹ eine Einheit der Formel (a), (b) oder (c): 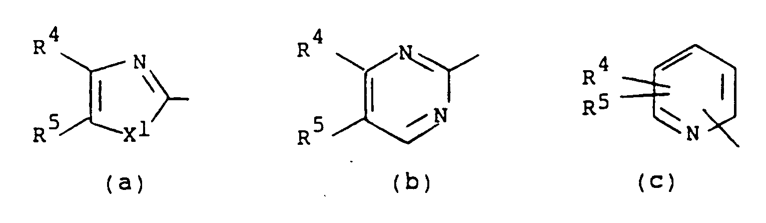 darstellt, wobei:
R⁴ und R⁵ jeweils unabhängig ein Wasserstoffatom, einen Alkyl- oder substituierten oder unsubstituierten Arylrest darstellen, oder, wenn R⁴ und R⁵ jeweils an benachbarte Kohlenstoffatome gebunden sind, dann R⁴ und R⁵ zusammen mit den Kohlenstoffatomen, an die sie gebunden sind, einen Benzolring bilden, wobei jedes durch R⁴ und R⁵ zusammen dargestellte Kohlenstoffatom substituiert oder unsubstituiert sein kann, und in der Einheit der Formel (a) X¹ ein Sauerstoff- oder Schwefelatom darstellt.

5. Verfahren nach Anspruch 4 zur Herstellung einer Verbindung, in der R⁴ und R⁵ zusammen eine Einheit der Formel (d): 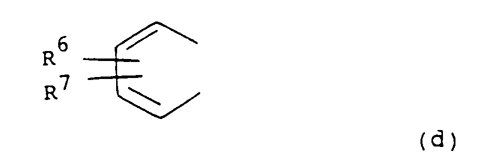 darstellen, in der R⁶ und R⁷ jeweils unabhängig ein Wasserstoff-, Halogenatom, einen substituierten oder unsubstituierten Alkyl- oder Alkoxyrest darstellen.

6. Verfahren nach einem der Ansprüche 1 bis 5 zur Herstellung einer Verbindung, in der A² eine Einheit der Formel (e): 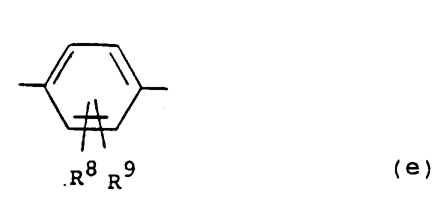 darstellt, in der R⁸ und R⁹ jeweils unabhängig ein Wasserstoff-, Halogenatom, einen substituierten oder unsubstituierten Alkyl- oder Alkoxyrest darstellen.

7. Verfahren nach einem der Ansprüche 1 bis 6 zur Herstellung einer Verbindung, in der Y ein Sauerstoffatom darstellt.

8. Verfahren nach Anspruch 1 zur Herstellung von 5-(4-[2-(N-Methyl-N-(2-benzoxazolyl)amino)ethoxy]benzyl)-5-methyl-2,4-thiazolidindion oder einer tautomeren Form davon und/oder eines pharmazeutisch verträglichen Salzes davon und/oder eines pharmazeutisch verträglichen Solvats davon.

9. Verwendung einer Verbindung der Formel (I) nach Anspruch 1 oder einer tautomeren Form davon und/oder eines pharmazeutisch verträglichen Salzes davon und/oder eines pharmazeutisch verträglichen Solvats davon zur Herstellung eines Medikaments zur Behandlung und/oder Prophylaxe von Hyperglykämie.

10. Verwendung einer Verbindung der Formel (I) nach Anspruch 1 oder einer tautomeren Form davon und/oder eines pharmazeutisch verträglichen Salzes davon und/oder eines pharmazeutisch verträglichen Solvats davon, zur Herstellung eines Medikaments zur Behandlung und/oder Prophylaxe von Hyperlipämie, Bluthochdruck oder Herzkreislauferkrankungen.

## Revendications (Revendications pour l'(les) Etat(s) contractant(s) suivant(s): AT, BE, CH, DE, DK, FR, GB, GR, IT, LI, LU, NL, SE)

1. Composé de formule (I) : 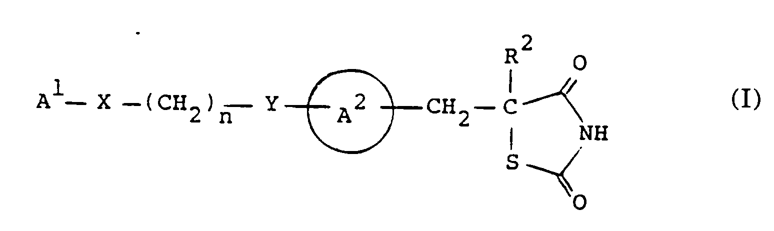 ou forme tautomère de celui-ci et/ou sel de celui-ci acceptable du point de vue pharmaceutique, et/ou solvate de ceux-ci acceptable du point de vue pharmaceutique, caractérisé en ce que :
A¹ représente un groupe aromatique hétérocyclyle substitué ou non substitué;
A² représente un cycle benzène portant au total jusqu'à cinq substituants;
X représente un atome d'oxygène, de soufre ou un groupe NR¹ dans lequel R¹ représente un atome d'hydrogène, un groupe alkyle, un groupe acyle, un groupe aralkyle dans lequel la partie aryle peut être substituée ou non substituée, ou un groupe aryle substitué ou non substitué;
Y représente un atome d'oxygène ou de soufre;
R² représente un groupe alkyle, aralkyle ou aryle; et
n représente un entier dans la gamme de 2 à 6.

2. Composé suivant la revendication 1, dans lequel R² représente un groupe alkyle.

3. Composé suivant les revendications 1 ou 2, dans lequel R² représente un groupe méthyle.

4. Composé suivant l'une quelconque des revendications 1 à 3, dans lequel A¹ représente une partie de formules (a), (b) ou (c) : 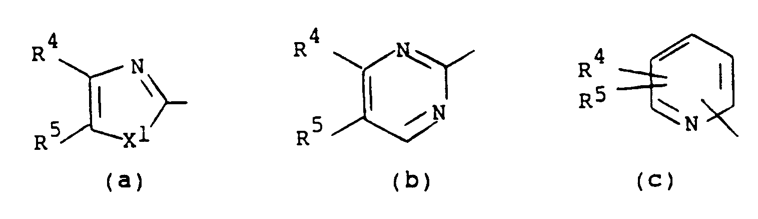 dans lesquelles :
R⁴ et R⁵ sont indépendamment un atome d'hydrogène, un groupe alkyle ou un groupe aryle substitué ou non substitué, ou lorsque R⁴ et R⁵ sont chacun attachés à des atomes de carbone adjacents, R⁴ et R⁵ forment alors avec les atomes de carbone auxquels ils sont attachés, un cycle benzène dans lequel chaque atome de carbone représenté par R⁴ et R⁵ ensemble peut être substitué ou non substitué; et dans la partie de formule (a), X¹ représente un atome d'oxygène ou de soufre.

5. Composé suivant la revendication 4, dans lequel R⁴ et R⁵ forment ensemble une partie de formule (d) : 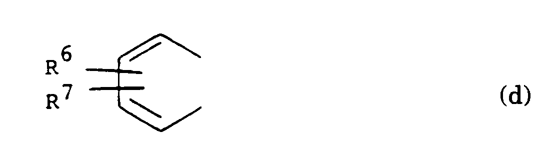 dans laquelle R⁶ et R⁷ représentent chacun indépendamment un atome d'hydrogène, d'halogène, un groupe alkyle ou alcoxy substitué ou non substitué.

6. Composé suivant l'une quelconque des revendications 1 à 5, dans lequel A² représente une partie de formule (e) : 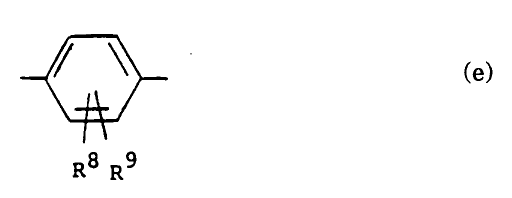 dans laquelle R⁸ et R⁹ représentent chacun indépendamment un atome d'hydrogène, d'halogène, un groupe alkyle ou alcoxy substitué ou non substitué.

7. Composé suivant l'une quelconque des revendications 1 à 6, dans lequel Y représente un atome d'oxygène.

8. Composé suivant la revendication 1, qui est la 5-(4-[2-(N-méthyl-N-(2-benzoxazolyl)-amino)-éthoxy]-benzyl)-5-méthyl-2,4-thiazolidinedione, une forme tautomère de celle-ci et/ou un sel de celle-ci acceptable du point de vue pharmaceutique, et/ou un solvate de ceux-ci acceptable du point de vue pharmaceutique.

9. Procédé pour la préparation d'un composé de formule (I) ou d'une forme tautomère de celui-ci et/ou d'un sel de celui-ci acceptable du point de vue pharmaceutique, et/ou un solvate de ceux-ci acceptable du point de vue pharmaceutique, caractérisé en ce que le procédé comprend :
(i) la réaction d'un composé de formule (III) : 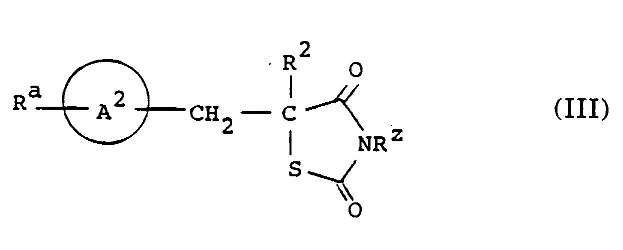 dans laquelle R² et A² sont tels que définis en relation avec la formule (I) dans la revendication 1, R^{z} est un atome d'hydrogène ou un groupe protégeant l'atome d'azote et R^{a} est une partie pouvant être convertie en une partie de formule (f) :
A¹-X-(CH₂)ₙ-Y- (f)
dans laquelle A¹, X, Y et n sont tels que définis en relation avec la formule (I), avec un réactif approprié capable de convertir R^{a} en cette partie (f);
(ii) la réaction d'un composé de formule (XII) : 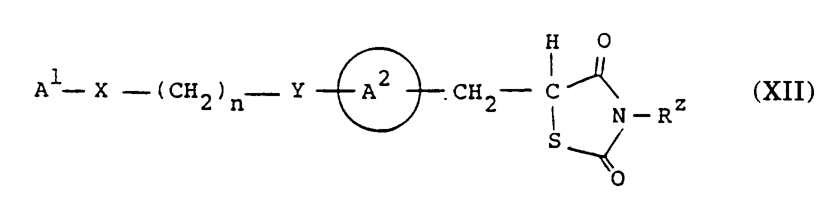 dans laquelle A¹, A², X, Y et n sont tels que définis en relation avec la formule (I) dans la revendication 1, et R^{z} est tel que défini en relation avec la formule (III) dans la réaction (i) ci-dessus, avec un composé de formule (VI) :
R² - R^{c} (VI)
dans laquelle R² est tel que défini en relation avec la formule (I) et R^{c} est un groupe mobile; ou
(iii) la réaction d'un composé de formule (XVI) : 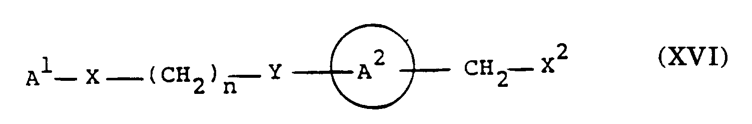 dans laquelle A¹, A², X, Y et n sont tels que définis en relation avec la formule (I) dans la revendication 1, et X² est un atome d'halogène, avec un composé de formule (X) : 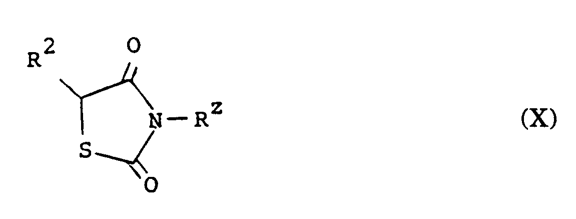 dans laquelle R² est tel que définis en relation avec la formule (I) dans la revendication 1 et R^{z} est tel que défini en relation avec la formule (III) dans la réaction (i) ci-dessus;
et ensuite, si cela est requis, la réalisation d'une ou de plusieurs étapes facultatives suivantes :
(i) conversion d'un composé de formule (I) en un autre composé de formule (I);
(ii) élimination d'un quelconque groupe protecteur;
(iii) préparation d'un sel acceptable du point de vue pharmaceutique d'un composé de formule (I) et/ou d'un solvate de ceux-ci acceptable du point de vue pharmaceutique.

10. Composition pharmaceutique comprenant un composé de formule (I) suivant la revendication 1, ou une forme tautomère de celui-ci ou un sel de celui-ci acceptable du point de vue pharmaceutique, ou un solvate de ceux-ci acceptable du point de vue pharmaceutique, et un support acceptable du point de vue pharmaceutique pour celui-ci.

11. Composé de formule (I) suivant la revendication 1, ou forme tautomère de celui-ci et/ou sel de celui-ci acceptable du point de vue pharmaceutique, et/ou solvate de ceux-ci acceptable du point de vue pharmaceutique, utile comme substance thérapeutique active.

12. Composé de formule (I) suivant la revendication 1, ou forme tautomère de celui-ci et/ou sel de celui-ci acceptable du point de vue pharmaceutique, et/ou solvate de ceux-ci acceptable du point de vue pharmaceutique, utile dans le traitement et/ou la prophylaxie de l'hyperglycémie.

13. Composé de formule (I) suivant la revendication 1, ou forme tautomère de celui-ci et/ou sel de celui-ci acceptable du point de vue pharmaceutique, et/ou solvate de ceux-ci acceptable du point de vue pharmaceutique, utile dans le traitement et/ou la prophylaxie de l'hyperlipidémie et/ou de l'hypertension et/ou d'une affection cardiovasculaire.

14. Utilisation d'un composé de formule (I) suivant la revendication 1, ou d'une forme tautomère de celui-ci et/ou d'un sel de celui-ci acceptable du point de vue pharmaceutique, et/ou d'un solvate de ceux-ci acceptable du point de vue pharmaceutique, pour la fabrication d'un médicament pour le traitement et/ou la prophylaxie de l'hyperglycémie.

15. Utilisation d'un composé de formule (I) suivant la revendication 1, ou d'une forme tautomère de celui-ci et/ou d'un sel de celui-ci acceptable du point de vue pharmaceutique, et/ou d'un solvate de ceux-ci acceptable du point de vue pharmaceutique, pour la fabrication d'un médicament pour le traitement et/ou la prophylaxie de l'hyperlipidémie et/ou de l'hypertension et/ou d'une affection cardiovasculaire.

## Revendications (Revendications pour l'(les) Etat(s) contractant(s) suivant(s): ES)

1. Procédé pour la préparation d'un composé de formule (I) : 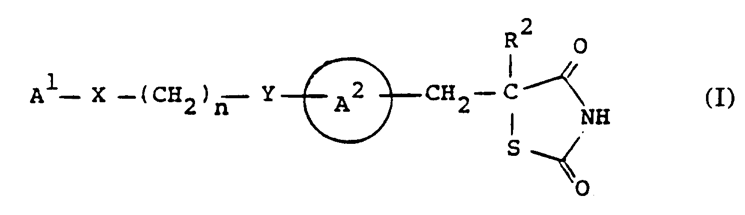 ou d'une forme tautomère de celui-ci et/ou d'un sel de celui-ci acceptable du point de vue pharmaceutique, et/ou d'un solvate de ceux-ci acceptable du point de vue pharmaceutique, dans laquelle :
A¹ représente un groupe aromatique hétérocyclyle substitué ou non substitué;
A² représente un cycle benzène portant au total jusqu'à cinq substituants;
X représente un atome d'oxygène, de soufre ou un groupe NR¹ dans lequel R¹ représente un atome d'hydrogène, un groupe alkyle, un groupe acyle, un groupe aralkyle dans lequel la partie aryle peut être substituée ou non substituée, ou un groupe aryle substitué ou non substitué;
Y représente un atome d'oxygène ou de soufre;
R² représente un groupe alkyle, aralkyle ou aryle; et
n représente un entier dans la gamme de 2 à 6,
caractérisé en ce que le procédé comprend :
(i) la réaction d'un composé de formule (III) : 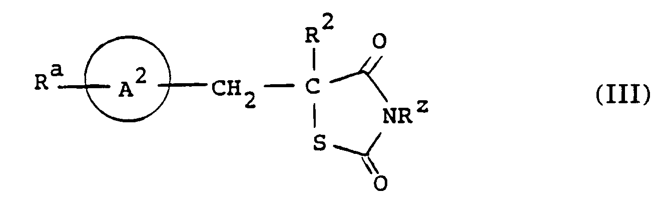 dans laquelle R² et A² sont tels que définis en relation avec la formule (I) dans la revendication 1, R^{z} est un atome d'hydrogène ou un groupe protégeant l'atome d'azote et R^{a} est une partie pouvant être convertie en une partie de formule (f) :
A¹-X-(CH₂)ₙ-Y- (f)
dans laquelle A¹, X, Y et n sont tels que définis en relation avec la formule (I), avec un réactif approprié capable de convertir R^{a} en cette partie (f);
(ii) la réaction d'un composé de formule (XII) : 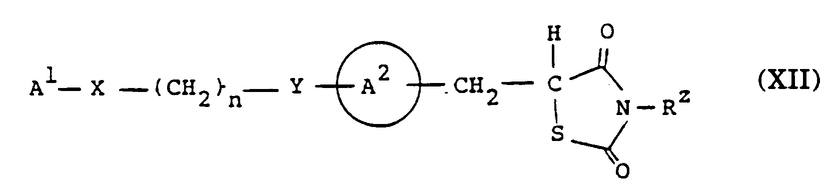 dans laquelle A¹, A², X, Y et n sont tels que définis en relation avec la formule (I) dans la revendication 1, et R^{z} est tel que défini en relation avec la formule (III) dans la réaction (i) ci-dessus, avec un composé de formule (VI) :
R² - R^{c} (VI)
dans laquelle R² est tel que défini en relation avec la formule (I) et R^{c} est un groupe mobile; ou
(iii) la réaction d'un composé de formule (XVI) : 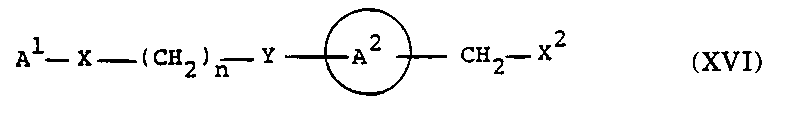 dans laquelle A¹, A², X, Y et n sont tels que définis en relation avec la formule (I) dans la revendication 1, et X² est un atome d'halogène, avec un composé de formule (X) : 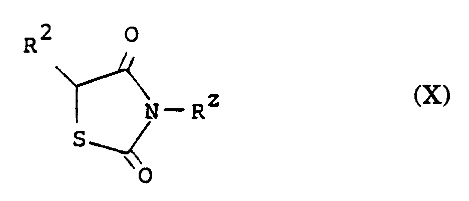 dans laquelle R² est tel que définis en relation avec la formule (I) dans la revendication 1 et R^{z} est tel que défini en relation avec la formule (III) dans la réaction (i) ci-dessus;
et ensuite, si cela est requis, la réalisation d'une ou de plusieurs étapes facultatives suivantes :
(i) conversion d'un composé de formule (I) en un autre composé de formule (I);
(ii) élimination d'un quelconque groupe protecteur;
(iii) préparation d'un sel acceptable du point de vue pharmaceutique d'un composé de formule (I) et/ou d'un solvate de ceux-ci acceptable du point de vue pharmaceutique.

2. Procédé suivant la revendication 1, pour préparer un composé dans lequel R² représente un groupe alkyle.

3. Procédé suivant les revendications 1 ou 2, pour préparer un composé dans lequel R² représente un groupe méthyle.

4. Procédé suivant l'une quelconque des revendications 1 à 3, pour préparer un composé dans lequel A¹ représente une partie de formules (a), (b) ou (c) : 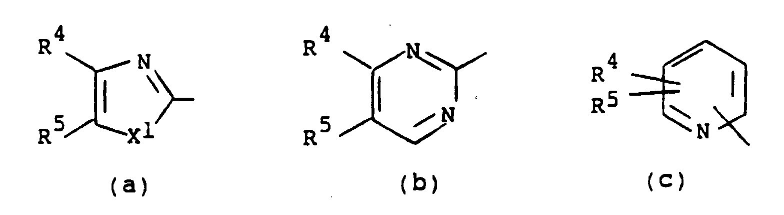 dans lesquelles :
R⁴ et R⁵ sont indépendamment un atome d'hydrogène, un groupe alkyle ou un groupe aryle substitué ou non substitué, ou lorsque R⁴ et R⁵ sont chacun attachés à des atomes de carbone adjacents, R⁴ et R⁵ forment alors avec les atomes de carbone auxquels ils sont attachés, un cycle benzène dans lequel chaque atome de carbone représenté par R⁴ et R⁵ ensemble peut être substitué ou non substitué; et dans la partie de formule (a), X¹ représente un atome d'oxygène ou de soufre.

5. Procédé suivant la revendication 4, pour préparer un composé dans lequel R⁴ et R⁵ forment ensemble une partie de formule (d) : 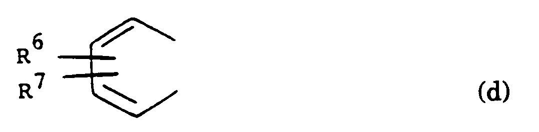 dans laquelle R⁶ et R⁷ représentent chacun indépendamment un atome d'hydrogène, d'halogène, un groupe alkyle ou alcoxy substitué ou non substitué.

6. Procédé suivant l'une quelconque des revendications 1 à 5, pour préparer un composé dans lequel A² représente une partie de formule (e) : 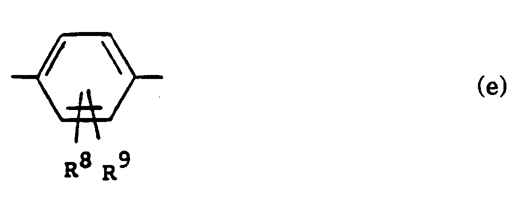 dans laquelle R⁸ et R⁹ représentent chacun indépendamment un atome d'hydrogène, d'halogène, un groupe alkyle ou alcoxy substitué ou non substitué.

7. Procédé suivant l'une quelconque des revendications 1 à 6, pour préparer un composé dans lequel Y représente un atome d'oxygène.

8. Procédé suivant la revendication 1, pour préparer la 5-(4-[2-(N-méthyl-N-(2-benzoxazolyl)-amino)-éthoxy]-benzyl)-5-méthyl-2,4-thiazolidinedione, une forme tautomère de celle-ci et/ou un sel de celle-ci acceptable du point de vue pharmaceutique, et/ou un solvate de ceux-ci acceptable du point de vue pharmaceutique.

9. Utilisation d'un composé de formule (I) suivant la revendication 1, ou d'une forme tautomère de celui-ci et/ou d'un sel de celui-ci acceptable du point de vue pharmaceutique, et/ou d'un solvate de ceux-ci acceptable du point de vue pharmaceutique, pour la fabrication d'un médicament pour le traitement et/ou la prophylaxie de l'hyperglycémie.

10. Utilisation d'un composé de formule (I) suivant la revendication 1, ou d'une forme tautomère de celui-ci et/ou d'un sel de celui-ci acceptable du point de vue pharmaceutique, et/ou d'un solvate de ceux-ci acceptable du point de vue pharmaceutique, pour la fabrication d'un médicament pour le traitement et/ou la prophylaxie de l'hyperlipidémie et/ou de l'hypertension et/ou d'une affection cardiovasculaire.
